# EUROPEAN PATENT APPLICATION

(11) **EP 4 219 722 A1**
(43) Date of publication of application: **02.08.2023**
(21) Application number: 21872944.0
(22) Date of filing: 24.09.2021
(51) Int. Cl.: C12N 15/74

(54) **EXPRESSION VECTOR FOR USE IN METHANOTROPH**

(30) Priority: 25.09.2020 KR 20200125080; 17.09.2021 KR 20210124777
(71) Applicant: Korea Research Institute of Bioscience and Biotechnology, Daejeon 34141 (KR)
(72) Inventor: LEE, Seung Goo, Daejeon 34141 (KR); LEE, Dae Hee, Daejeon 34141 (KR); LEE, Hye Won, Daejeon 34141 (KR); KIM, Seong Keun, Daejeon 34141 (KR); BAEK, Ji In, Daejeon 34141 (KR); KIM, Tae Hyun, Daejeon 34141 (KR); OH, So Hyung, Daejeon 34141 (KR)
(74) Representative: Cabinet Laurent & Charras
(86) International application number: PCT/KR2021/013043
(87) International publication number: WO 2022/065916

(57) **Abstract**

Provided is a vector comprising: a first expression cassette comprising DmpR as a transcriptional control gene, a ribosome binding site, and a promoter operably linked to same; a second expression cassette comprising a sequence encoding a target polypeptide, a ribosome binding site, and a Pₒ promoter operably linked to same. Also provided is a transformed methanotroph transformed by said vector.

## Description

### BACKGROUND OF THE INVENTION

### Field of the Invention

The disclosure relates to expression vectors for use in methanotrophs.

### Description of the Related Art

Methane, which is the main component of natural gas, shale gas and biogas, is a greenhouse gas that needs be reduced. However, it is also a promising next-generation carbon resource for bio-industrial applications. Traditional chemical methane conversion methods are inefficient due to the multi-step process involved and high temperature/high pressure requirements.

To overcome these disadvantages, a biological conversion method has recently been developed. Methanotrophs, which use methane as their carbon source, can convert methane at room temperature and normal pressure using methane monooxygenase. This opens up the possibility of producing various high-value biochemicals from methane based on engineering metabolic pathway of methanotrophs.

However, despite this possibility, in the case of transforming methanotrophs using previously known molecular biological tools (e.g., vectors, expression cassettes, and promoters) for gene expression, metabolism control, or the like, expected effects are often not achieved. Therefore, methanotrophs are not actively utilized industrially. In particular, existing methanotrophs expression systems mainly depend on a constitutive promoter, and in the methanotrophs transformed with an expression system having an inducible promoter, there is a problem in that the expression of the target protein is uneven between cells and the expression level is not sufficient.

### SUMMARY OF THE INVENTION

One embodiment provides a vector that can be used for expressing a target polypeptide in methanotrophs, regulating or promoting the expression of target genes of methanotrophs to reach a desired expression level, or for other genetic manipulations of methanotrophs.

One aspect of the disclosure provides a vector for introduction into a methanotroph, comprising a first expression cassette comprising DmpR as a transcriptional regulator and a promoter operably linked thereto; and a second expression cassette comprising a sequence encoding a target polypeptide and a Pₒ promoter operably linked thereto.

In one embodiment, the target polypeptide may be a marker, a reporter, a polypeptide capable of improving production of a desired metabolite, or a polypeptide capable of promoting growth of the methanotroph.

In one embodiment, in the vector, expression of the target polypeptide may be induced by a phenolic compound.

In one embodiment, transcription directions of the first expression cassette and the second expression cassette may be the same or opposite.

In one embodiment, the vector may be a plasmid.

In one embodiment, the methanotroph may be selected from the group consisting of *Methylomonas sp., Methylobacter sp., Methylococcus sp., Methylosphaera sp., Methylocaldum sp., Methyloglobus sp., Methylosarcina sp., Methyloprofundus sp., Methylothermus sp., Methylohalobius sp., Methylogaea sp., Methylomarinum sp., Methylovulum sp., Methylomarinovum sp., Methylorubrum sp., Methyloparacoccus sp., Methylosinus sp., Methylocystis sp., Methylocella sp., Methylocapsa sp., Methylofurula sp., Methylacidiphilum sp., Methylacidimicrobium sp.,* and Methylomicrobium sp.

In one embodiment, the methanotroph may be *Methylococcus capsulatus* Bath or *Methylosinus trichosporium* OB3b.

Another aspect of the disclosure provides a methanotroph in which the above vector is introduced.

The expression vector according to one embodiment can precisely regulate the timing and amount of a specific gene expression in methanotrophs by controlling the concentration or timing of inducer addition.

Methanotrophs into which the expression vector according to one embodiment is introduced show a homogenous expression level among individual cells, facilitating research based on various high-throughput large-scale analysis techniques by utilizing flow cytometry and microscopy. This enhances the speed and efficiency of methanotrophs research.

Since the methanotrophs into which the expression vector according to one embodiment is introduced show excellent expression of the target protein, they can be used effectively for large-scale production of recombinant proteins using methane gas.

The expression vector according to one embodiment can be used for metabolic engineering or synthetic biology applications that are based on methanotrophs.

The vector according to one embodiment can be used for gene transfer and enhancing the efficiency of metabolic engineering in methanotrophs, as well as regulating target gene expression or increasing target gene expression.

### BRIEF DESCRIPTION OF THE DRAWINGS

FIG. 1 shows a vector map of a pAWP89 plasmid.
FIG. 2 shows an expression cassette and its inducers according to an embodiment.
FIG. 3 shows the expression level of dTomato in M. *trichosporium* OB3b, into which a vector containing a TetR-Pₜₑₜ-dTomato expression cassette is introduced, as determined by varying concentrations of an anhydrotetracycline (aTc) inducer and over time. (A) shows the measurements of the average fluorescence value/OD₆₀₀ using a microplate reader, while (B) shows the distribution of fluorescent expressions of individual cells measured by a flow cytometer.
FIG. 4 shows the expression level of dTomato in *M. capsulatus* Bath, into which a vector containing a DmpR-Pₒ-dTomato, LacI-P_{trc}-dTomato, or XylS-Pₘ-dTomato expression cassette is introduced, as determined by measuring the average fluorescence value/OD600 at varying concentrations of an inducer .
FIG. 5 shows the distribution of dTomato expression levels in *M. capsulatus* Bath, into which a vector containing a LacI-P_{trc}-dTomato, XylS-Pₘ-dTomato or DmpR-Pₒ-dTomato expression cassette is introduced, as determined by measuring fluorescence intensities of individual cells at varying concentrations of an inducer.
FIG. 6 represents the expression level of dTomato in *M. capsulatus* Bath, into which a vector containing a DmpR-Pₒ-dTomato expression cassette is introduced, as analyzed by SDS-PAGE.
FIG. 7 shows the difference in dTomato expression levels between wild-type M. *capsulatus* Bath and recombinant M. *capsulatus* Bath in which a vector containing a DmpR-Pₒ-dTomato expression cassette is introduced, under conditions with 10 µM phenol and without phenol.
FIG. 8 represents the time-dependent expression level of dTomato in *M. capsulatus* Bath upon phenol induction at a concentration of 10 µM, using a vector containing a DmpR-Pₒ-dTomato expression cassette.
FIG. 9 shows the dose-dependent expression level of dTomato in *M. trichosporium* OB3b upon induction with different concentrations of inducers, using a vector containing a DmpR-Pₒ-dTomato, LacI-P_{trc}-dTomato, or XylS-Pₘ-dTomato expression cassette, measured as an average fluorescence value/OD₆₀₀.
FIG. 10 shows the distribution of dTomato expression levels in individual cells of *M. trichosporium* OB3b upon induction with different concentrations of inducers, using a vector containing a LacI-P_{trc}-dTomato, XylS-Pₘ-dTornato or DmpR-Pₒ-dTomato expression cassette, measured as fluorescence intensities.
FIG. 11 shows an expression level of dTomato in *M. trichosporium* OB3b in which a vector containing a DmpR-Pₒ-dTomato expression cassette is introduced, as analyzed by SDS-PAGE.
FIG. 12 shows the induced expression level of dTomato in *M. capsulatus* Bath, into which a vector containing a DmpR-Pₒ-dTomato expression cassette is introduced, under sMMO or pMMO expression conditions, upon supplementation with benzene that can be converted into phenol by sMMO.
FIG. 13 depicts a diagram illustrating the improvement of an expression levels for methanotrophs based on different ribosome binding sites.
FIG. 14 shows the gene expression levels in *M. capsulatus* Bath containing a pAWP78 vector, a FmDTA-pAWP or Pₒ-FmDTA-pAWP expression cassette, as determined by the presence of absence of phenol.
FIG. 15 depicts a diagram of a CRISPR/Cas9-based cytosine base editor plasmid construction, along with the conversion rate of point mutations from cytosine to thymine using the constructed plasmid.

### DETAILED DESCRIPTION OF THE INVENTION

One aspect provides a vector for introduction into a methanotroph, comprising a first expression cassette containing DmpR as a transcriptional regulator and a promoter operably linked thereto; and a second expression cassette containing a sequence encoding a target polypeptide and a Pₒ promoter operably linked thereto.

The vector may be used for artificially manipulating the metabolic process of methanotrophs such as heterologous expression in methanotrophs according to the type of sequence encoding a target polypeptide contained therein, or regulation or promotion of the production of endogenous proteins. The vector may be for artificially regulating the metabolic process of methanotrophs or constructing a new metabolic circuit. The vector may be for increasing or decreasing the productivity of an endogenous substance. The vector may be for imparting new substance production capability. The vector may be for increasing or controlling the growth rate of methanotrophs. The vector may be for transforming methanotrophs. The term "transformation" means introducing a vector containing a polynucleotide encoding a target polypeptide into a host cell so that a protein encoded by the polynucleotide can be expressed in the host cell.

The term "operably linked" means that DNA regions are functionally related to each other. For example, In the case that a promoter regulates transcription of a gene sequence, a promoter and a gene sequence are operably linked.

The methanotrophs are strains that can grow using methane as a carbon source at room temperature and under normal pressure using methane monooxygenase(MMO). The methane monooxygenase is classified into soluble methane monooxygenase(sMMO) expressed in the cytoplasm and particulate methane monooxygenase(pMMO) expressed in the cell membrane. The MMO oxidizes methane to methanol, and methanol is converted to formaldehyde and then enters the serine pathway and/or RuMP pathway depending on the type of methanotrophs.

The di-methyl phenol regulator(DmpR) is a transcription factor for a phenol degrading protein, and may be derived from a microorganism of *Pseudomonas sp.* The DmpR exists as an unreactive dimer, but when combined with phenol, it changes to a polymer such as a tetramer or hexamer in which four molecules are bonded. The DmpR polymers formed by the reaction with phenol may bind to a Pₒ promoter and express a target polypeptide operably linked to the Pₒ promoter. According to one embodiment, the expression of dTomato in methanotrophs in which a vector containing DmpR is introduced is higher than that in methanotrophs in which a vector containing TetR, LacI, or XylS is contained as a regulator.

The sequence of the DmpR gene may consist of the nucleotide sequence of SEQ ID NO: 1. The DmpR, which consists of the above SEQ ID NO: 1, may include a polynucleotide including nucleotide sequence that has at least 60 % or more, 70 % or more, 80 % or more, 83 % or more, 84 % or more, 88 % or more, 90 % or more, 93 % or more, 95 % or more, or 97 % or more homology to the nucleotide sequence of SEQ ID NO: 1. Any polynucleotide sequence with deletion, modification, substitution, or addition in part of the sequence may also be included within the scope of the disclosure, as long as the sequence has the homology to the above sequence and exhibits biological activity substantially identical to or corresponding to that of the above sequence.

As the transcriptional regulator, a gene encoding a DmpR variant may be used instead of wild-type DmpR. The DmpR variant may be a variant with improved phenol sensitivity and substrate specificity. For example, the variant may be one in which the 71st glutamine (Q) is substituted with alanine (A), the 135th glutamic acid (E) is substituted with lysine (K), or the 188th lysine (K) is substituted with arginine (R) in the wild-type DmpR consisting of the amino acid sequence of SEQ ID NO: 35. Specifically, the DmpR variant may include one or more mutations selected from the group consisting of M52I, M52C, Q71A, E135K, and K188R. For example, the DmpR variant may be DmpR(M52I), DmpR(M52C), DmpR(M52I/K188R), DmpR(M52I/Q71A) or DmpR(M52C/E135K). When the gene encoding the DmpR variant is introduced as a transcriptional regulator, phenol sensitivity and substrate specificity can be improved compared to using the wild-type DmpR gene.

The DmpR variant may be a DmpR variant capable of inducing constitutive expression by forming polymer even in the absence of phenol. Since this can activate expression by the Pₒ promoter, constitutive expression of the target polypeptide can also be achieved. The DmpR variant inducing constitutive expression may consist of the DmpR (Δ2-204) sequence of SEQ ID NO: 2, which is a sequence encoding DmpR (Δ2-204) in which 2^{nd} to 204^{th} amino acids of the wide DmpR are deleted.

The term "homology" may indicate the degree of matching with the given nucleotide sequence, and may be presented as a percentage (%). In the specification, a homology sequence having an activity which is identical or similar to the given nucleotide sequence is presented as "% homology". The homology to the nucleotide sequence can be determined by, for example, algorithm BLAST (see Karlin and Altschul, Pro. Natl. Acad. Sci. USA, 90, 5873, 1993) or FASTA (see Pearson, Methods Enzymol., 183, 63, 1990). Based on this algorithm BLAST, programs called BLASTN or BLASTX have been developed (see http://www.ncbi.nlm.nih.gov).

The Pₒ promoter can activate the transcription of a target polypeptide operably linked thereto by binding to the multimerized DmpR in reaction with phenol. Information on the Pₒ promoter may be found in ACS Synth Biol. 2014 Mar 21;3(3): 163-171. The sequence of the Pₒ promoter may consist of the nucleotide sequence of SEQ ID NO: 3. The Pₒ promoter, which consists of SEQ ID NO: 2, may include a polynucleotide including nucleotide sequence that has at least 60 % or more, 70 % or more, 80 % or more, 83 % or more, 84 % or more, 88 % or more, 90 % or more, 93 % or more, 95% or more, or 97% or more homology to the sequence of SEQ ID NO: 2. Any polynucleotide sequence with deletion, modification, substitution, or addition in part of the sequence may also be included within the scope of the disclosure, as long as the sequence has the homology to the above sequence and exhibits biological activity substantially identical to or corresponding to that of the above sequence.

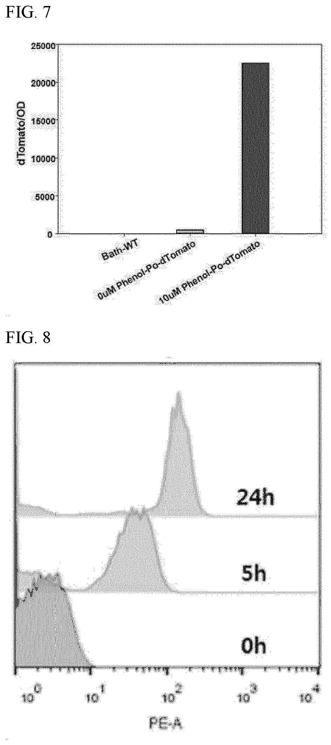

Since the vector can tightly regulate the inducible expression of the target polypeptide, there is an advantage in that there is no or very low leaky expression of the target polypeptide in the absence of an inducer. The leaky expression means a basal level expression that occurs in the absence of an inducer. According to FIG. 7, assuming that the vector has 100 % fluorescence by dTomato expression in the presence of 10 µM phenol, the rate of fluorescence by dTomato expression in the absence of phenol is maintained at a very low level of 2 % or less, resulting in excellent inhibition efficacy of leak expression.

The vector may further include a ribosomal binding site(RBS). The ribosomal binding site may also be referred to as a Shine-Dalgarno sequence. The ribosomal binding site may be located upstream of a target protein gene sequence or between a promoter gene sequence and a target protein sequence. A spacer sequence may be further included between the ribosomal binding site and the initiation codon of the target protein. The sequence of the ribosomal binding site may be a methanotroph-derived RBS sequence.

The sequence of the ribosomal binding site may consist of the nucleotide sequence of SEQ ID NO: 4. The nucleotide sequence of SEQ ID NO: 4 is a Bacteriophage T7 gene 10-derived sequence.

The ribosomal binding site sequence may be selected from the group consisting of the RBS sequence of mxaF(MDH) of SEQ ID NO: 5, the RBS sequence of *mmoX* of SEQ ID NO: 6, the RBS sequence of mmoY of SEQ ID NO: 7, the RBS sequence of *mmoZ* of SEQ ID NO: 8, the RBS sequence of pmoA1 of SEQ ID NO: 9, the RBS sequence of *pmoB1* of SEQ ID NO: 10, the RBS sequence of pmoC1 of SEQ ID NO: 11, the RBS sequence of *pmoC2* of SEQ ID NO: 12, and the RBS sequence of pmoC3 of SEQ ID NO: 13. A vector containing the ribosomal binding site sequence consisting of a sequence selected from the sequences of SEQ ID NOs: 6 to 13 can increase the expression of a target polypeptide in methanotrophs than that in a vector containing the RBS sequence of methanol dehydrogenase(MDH).

The RBS, which consists of the sequences of the above SEQ ID NOs: 4 to 13, may include a polynucleotide including nucleotide sequence that has at least 60 % or more, 70 % or more, 80 % or more, 83 % or more, 84 % or more, 88 % or more, 90 % or more, 93 % or more, 95 % or more, or 97 % or more homology to the respective nucleotide sequences. Any polynucleotide sequence with deletion, modification, substitution, or addition in part of the sequence may also be included within the scope of the disclosure, as long as the sequence has the homology to the above sequences and exhibits biological activity substantially identical to or corresponding to that of the above sequences.

The target polypeptide may be, for example, a marker or reporter. The target polypeptide may be for the production of a foreign protein. The target polypeptide may be for improving the production of a desired metabolite, promoting the growth of methanotrophs, producing a specific protein, changing or inhibiting the production of metabolites of methanotrophs, or inhibiting the expression or activity of a specific enzyme. The target polypeptide may be, for example, formate dehydrogenase(FDH), particulate methane monooxygenase(pMMO), threonine aldolase(TA), isoprene synthase, or lactate dehydrogenase, but is not limited thereto. The marker or reporter protein may be a fluorescent protein or an antibiotic resistance gene protein, for example, a fluorescent protein such as dTomato, but is not particularly limited. The dTomato may consist of the nucleotide sequence of SEQ ID NO: 14.

The sequence encoding the target polypeptide may be a codon-optimized sequence for the methanotroph to introduce the sequence.

The vector may contain multiple cloning sites for introducing the sequence encoding the target polypeptide, in place of the sequence. The multiple cloning sites are sites into which genes encoding a target polypeptide or target protein to be expressed through the expression vector of the disclosure are inserted.

According to one embodiment, the vector may induce the expression of the target polypeptide by a phenolic compound. Since sMMO of methanotrophs may oxidize benzene to phenol, the vector may activate the expression of the target protein even by benzene. According to one embodiment, the present inventors confirmed that phenol was superior to other expression inducers such as aTc and IPTG in inducing the expression of methanotrophs. Compared to aTc, IPTG, and benzoate, phenol has a relatively small molecular weight and size, and is more hydrophilic or amphipathic, so it may be easy to activate the expression of the inducible vector inside the strain by penetrating, absorbing, or being received by methanotrophs. In addition, there is an advantage in that homogenous expression can be achieved because there is little difference in expression level between methanotroph cells. According to one embodiment, methanotrophs that induced expression by phenol showed significantly higher dTomato expression and a low and uniform intercellular distribution despite the use of a lower amount of phenol, compared to those that induced expression by anhydrotetracycline(aTc), IPTG, and benzoate. This may be due to the difference in the receptibility of methanotrophs to the inducer. The phenolic compound may include phenol, 2-chlorophenol, 2-iodophenol, o-cresol, 2-ethylphenol, m-cresol, 2-nitrophenol, catechol, 2-methoxyphenol, 2-aminophenol, 2,3-dichlorophenol, 3-chlorophenol, 2,3-dimethylphenol, 3-nitrophenol, 4-chlorophenol, p-cresol, 2,5-dichlorophenol, 2,5-dimethylphenol, or salicylic acid.

The expression cassette refers to a region that includes a coding sequence of a specific gene and a sequence regulating its expression. For example, the expression cassette may include a polynucleotide encoding a target polypeptide, a promoter operably linked thereto, a transcription termination signal, a ribosomal binding site, and a translation termination signal.

According to one embodiment, the transcription directions of the first expression cassette and second expression cassette may be the same or opposite. Reversely aligning the transcriptional directions may be advantageous for precise regulation of expression levels.

The vector may be a plasmid. Specifically, the plasmid may be a plasmid capable of delivery by conjugation to methanotrophs and replication in methanotrophs. For example, the plasmid may be a pBR-based, pUC-based, pBluescriptII-based, pGEM-based, pTZ-based, pCL-based or pET-based plasmid. Specifically, the plasmid may include pDZ, pACYC177, pACYC184, pCL, pECCG117, pUC19, pBR322, pMW118, pCC1BAC, pUC18, pBAD, pJK001, pCM, or pAWP plasmid, preferably pAWP, but is not particularly limited. According to one embodiment, the vector of the disclosure may be constructed by introducing the DmpR and the Pₒ promoter into the pAWP89 vector. The pAWP89 vector may consist of the sequence of SEQ ID NO: 15. The pAWP89 plasmid may be prepared or purchased commercially, and for example, the one from addgene's catalog #61264 may be used.

The methanotrophs may be selected from the group consisting *of Methylomonas sp., Methylobacter sp., Methylococcus sp., Methylosphaera sp.*, *Methylocaldum sp., Methyloglobus sp., Methylosarcina sp.*, *Methyloprofundus sp., Methylothermus sp., Methylohalobius sp., Methylogaea sp., Methylomarinum sp., Methylovulum sp., Methylomarinovum sp., Methylorubrum sp., Methyloparacoccus sp., Methylosinus sp., Methylocystis sp., Methylocella sp., Methylocapsa sp., Methylofurula sp., Methylacidiphilum sp.*, *Methylacidimicrobium sp.,* and *Methylomicrobium sp.,* and more specifically, *Methylococcus sp.* or *Methylosinus sp.*

The methanotrophs may be gamma-proteobacteria (also referred to as Group I), alpha-proteobacteria (also referred to as Group II), or verrucomicrobia (also referred to as Group III). Among them, gamma-proteobacteria are strains that use the RuMP pathway as a main metabolic pathway, and alpha-proteobacteria are strains that use the serine pathway as a main metabolic pathway.

The methanotrophs may be *Methylococcus capsulatus* Bath or *Methylosinus trichosporium* OB3b.

The expression vector may be an inducible expression vector. An inducible expression vector is introduced into methanotrophs, and is cultured until a sufficient number of strains are secured. Then, the target polypeptide may be expressed by adding an inducer in the culture. This has the advantage of increasing the efficiency of the strain growth and the target protein production because it can induce the expression of a specific gene at a necessary time compared to the case of using a constitutive expression vector.

The vector may include a replication origin or an origin of replication (oriV) that functions in methanotrophs. The vector may further include an origin of replication that functions in bacteria other than methanotrophs. The non-methanogenic bacteria may be donor bacteria of conjugation. The oriV may be, for example, oriV_{RP4/RK2} and/or oriV_{ColE1}. The oriV_{RP4/RK2} may consist of the sequence of SEQ ID NO: 16. The oriV_{ColE1} may consist of the sequence of SEQ ID NO: 17.

The vector may include an origin of transfer(oriT) for conjugative transfer, and may include, for example, oriT_{RP4/RK2}. The oriT may consist of the sequence of SEQ ID NO: 18.

The vector may include a sequence encoding a protein activating the origin of replication or a replication initiation protein, for example, a sequence encoding a TrfA protein. The TrfA may consist of the sequence of SEQ ID NO: 19.

The vector may include a sequence for plasmid maintenance and conjugative transfer, and may include, for example, a traJ or traJ' gene sequence. The traJ may consist of the sequence of SEQ ID NO: 20. The traJ' may consist of the sequence of SEQ ID NO: 21.

The vector may include an antibiotic resistance gene, for example, a kanamycin resistance gene(KanR). The KanR may consist of the sequence of SEQ ID NO: 22.

Another aspect provides a methanotroph into which the above vector is introduced. The methanotroph introduced with the vector may express a heterologous polypeptide by expressing the target polypeptide of the vector, or express an endogenous polypeptide. Alternatively, as the amount or pathway of metabolism is regulated, the expression of a specific gene may be increased or inhibited, or the growth of methanotrophs may be promoted or inhibited.

The methanotroph that may be used for the introduction is the same as that described above.

The vector may be introduced by conjugation, heat shock, or electroporation. The conjugation is mainly used to transfer nucleic acids to methanotrophs. The conjugation involves mixing donor cells and recipient cells. A donor cell may be a cell containing the vector. The conjugation may be performed by further mixing helper cells. The conjugation may be a biparental mating conjugation or a triparental mating conjugation. A vector delivery method by conjugation of methanotrophs is known to those skilled in the art, and for example, reference may be made to information disclosed in Stolyar et al., 1995, Mikrobiologiya 64: 686-691.

Another aspect provides a method for producing a target substance including culturing the vector-introduced methanotroph; and collecting a target substance from the culture of the methanotroph.

The target substance may include the methanotroph itself, single cell protein, biopolymers produced by methanotrophs such as methanol, formaldehyde, PHA or PHB, or a heterologous protein produced by the expression of the target polypeptide contained in the vector. The methanotroph in which the vector is introduced may produce new proteins by the genes contained in the vector, and the expression of endogenous genes may be increased or decreased by regulating or promoting metabolism, or the growth of the methanotroph may be promoted or inhibited.

The culture may be performed by a batch culture method, a continuous culture method, or a fed-batch culture method, and specifically, it may be cultured continuously in a fed batch or repeated fed batch process.

The culture may be cultured in a liquid medium or a solid medium. The culture may use an NMS medium known to be used for culturing methanotrophs. The medium may include a C1 substrate as a carbon source. The C1 substrate refers to a molecule without a bond between carbon and carbon, and may be, for example, methane, methanol, formate, formaldehyde, methylated amine, methylthiol, or carbon monoxide. The C1 substrate may be cultured by supplying, for example, 10, 20, 30, 40, 50, 60, or 70 % methane gas based on a headspace. The medium may be a copper-deficient or copper-added medium for expressing sMMO or pMMO in methanotrophs, and specifically, CuCl₂ may be added.

The culture may be stirred at 150 rpm to 300 rpm, 180 rpm to 270 rpm, or 200 rpm to 250 rpm for smooth contact between methane and the strain. The incubation temperature may be 15 to 45 °C, 20 to 40 °C, or 25 to 35 °C, and may be appropriately adjusted according to the type of methanotrophs.

The production method may further include expressing the target polypeptide by contacting the cultured methanotroph with an inducer. An inducer for inducing expression of the target polypeptide may be added to the medium. The inducer may be phenol or a phenolic compound. For example, the medium may contain 1 to 100 µm, 5 to 10 µm, or 10 µm of phenol.

The obtained product may be collected by a method of centrifugation, filtration, extraction, spraying, drying, evaporation, precipitation, crystallization, electrophoresis, fractional dissolution (e.g., ammonium sulfate precipitation), chromatography (e.g., HPLC, ion exchange, affinity, hydrophobic, and size exclusion), or the like.

### [Mode for the Invention]

Hereinafter, one or more specific embodiments will be described in more detail through examples. However, these examples are intended to illustrate one or more specific embodiments, and the scope of the disclosure is not limited to these examples.

### Experimental Example 1: Production of inducible expression plasmid expressing dTomato

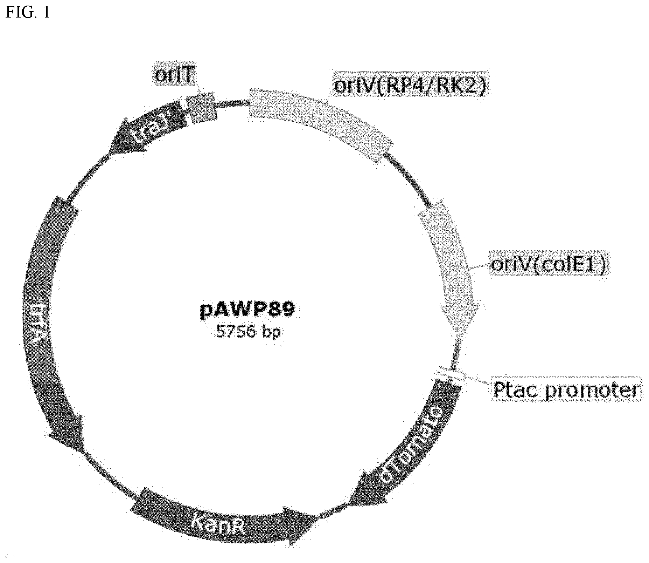

A pAWP89 vector was used. Details of the pAWP89 vector are described in Genetic Tools for the Industrially Promising Methanotroph Methylomicrobium buryatense (Appl Environ Microbiol. 2015 Mar;81(5):1775-1781). (FIG. 1)

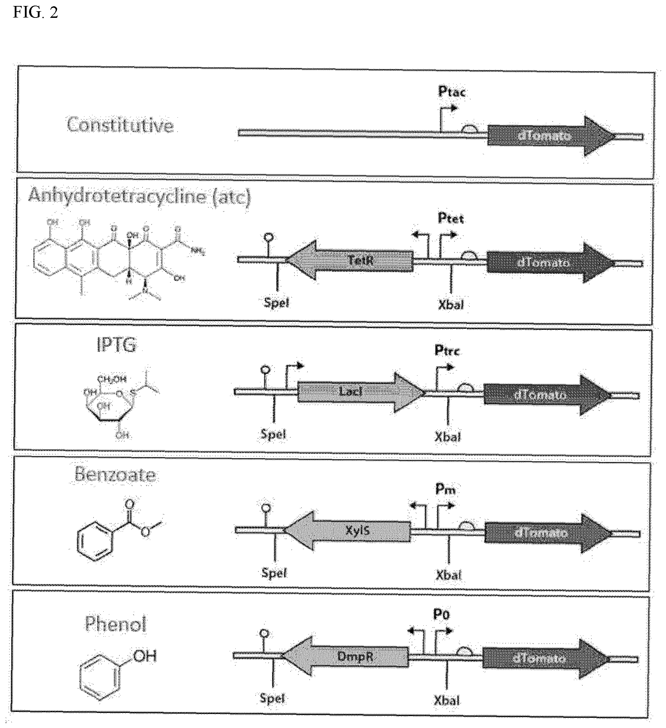

Plasmids were prepared by inserting TetR and Ptet, LacI and Ptrc, XylS and Pm, or DmpR and Po into the pAWP89 vector by the following method. (FIG. 2)

### 1-1. Construction of pAWP89-XbaI

In order to facilitate cloning of genes other than dTomato present in a pAWP89 plasmid (Addgene #61264), an XbaI restriction enzyme site was inserted while the dTomato gene was removed. First, the pAWP89 plasmid was digested with NotI/HindIII to prepare a DNA fragment. Then, a DNA fragment amplified using the pAWP89 plasmid as a template and a primer pair, pAW89(NotI)-F1 and pAW89(NotI)-R1, containing the XbaI restriction enzyme site, and a DNA fragment amplified using the pAWP89 plasmid as a template and a primer pair, pAW89(HindIII)-F2 and pAW89(HindIII)-R2, containing the XbaI restriction enzyme site were prepared, respectively. These three DNA fragments were combined by the Gibson assembly method to construct a pAWP89-XbaI plasmid into which the XbaI restriction enzyme site was inserted.

### 1-2. Construction of pAWP89S-XbaI plasmid

In order to facilitate promoter replacement while removing ColE1 origin of the pAWP89-XbaI plasmid, a SpeI restriction enzyme site was additionally inserted. Deleting the ColE1 origin reduces the size of the plasmid, increasing the efficiency of conjugation and preventing the growth inhibition of *E. coli,* a donor cell. First, the pAWP89-XbaI plasmid was digested with NotI/XbaI to prepare a DNA fragment. Then, a DNA fragment amplified using the pAWP89-XbaI plasmid as a template and a primer pair, pAWP89(NotI)-F1 and pAWPS(NotI)-R1, containing a SpeI restriction enzyme site, and a DNA fragment amplified using the pAWP89-XbaI plasmid as a template and a primer pair, pAWPS(XbaI)-F2 and pAWPS(XbaI)-R2, containing the SpeI restriction enzyme site, were prepared respectively. These three DNA fragments were combined by the Gibson assembly method to construct a pAWP89S-XbaI plasmid.

### 1-3. Construction of pAWP89S-Ptet-dTomato plasmid

To construct a plasmid expressing a dTomato protein by a Pₜₑₜ promoter, the pAWP89S-XbaI plasmid was digested with SpeI/XbaI to prepare a DNA fragment. Then, by performing amplification using pSEVA221-Ptet-dCas9 plasmid (see SEQ ID NO: 40) as a template and a primer pair, pTet-dTomato(SpeI)-F1 and pTet-dTomato(SpeI)-R1, a DNA fragment containing TetR and Ptet was prepared. Also, by performing amplification using the pAWP89 plasmid as a template and a primer pair, pTet-dTomato(XbaI)-F2 and pTet-dTomato(XbaI)-R2, a DNA fragment was prepared. These three DNA fragments were combined by the Gibson assembly method to construct a pAWP89S-Ptet-dTomato plasmid (see SEQ ID NO: 41).

### 1-4. Construction of pAWP89S-Ptrc-dTomato plasmid

In order to replace the Pₜₑₜ promoter of the pAWP89S-Pₜₑₜ-dTomato plasmid with the Ptrc promoter, the pAWP89S-Ptet-dTomato plasmid was digested with SpeI/XbaI to prepare a DNA fragment. Then, a DNA fragment containing LacI and Ptrc was prepared by performing amplification using a pSEVA234 plasmid (Silva-Rocha, Martinez-Garcia et al. 2013) as a template and a primer pair, pAWPS-Ptrc(SpeI)-F and pAWPS-Ptrc(XbaI)-R. The pSEVA234 plasmid may be sequenced from NCBI's GeneBank accession number (KC847292). These two DNA fragments were combined by the Gibson assembly method to construct a pAWP89S-Ptrc-dTomato plasmid (see SEQ ID NO: 42).

### 1-5. Construction of pAWP89S-Pₘ-dTomato Plasmid

In order to replace the Pₜₑₜ promoter of the pAWP89S-Pₜₑₜ-dTomato plasmid with the Pₘ promoter, the pAWP89S-Ptet-dTomato plasmid was digested with SpeI/Xbal to prepare a DNA fragment. Then, a DNA fragment containing XylS and Pm was prepared by performing amplification using the pSEVA228 plasmid (Silva-Rocha, Martinez-Garcia et al. 2013) as a template and a primer pair, pAWPS-Pₘ(SpeI)-F and pAWPS-Pm(XbaI)-R. The pSEVA228 plasmid may be sequenced from NCBI's GeneBank accession number (JX560388). These two DNA fragments were combined by the Gibson assembly method to construct a pAWP89S-Pₘ-dTomato plasmid (see SEQ ID NO: 43).

### 1-6. Construction of pAWP89S-Po-dTomato plasmid

In order to replace the Pₜₑₜ promoter of the pAWP89S-Pₜₑₜ-dTomato plasmid with the Pₒ promoter, the pAWP89S-Ptet-dTomato plasmid was digested with SpeI/Xbal to prepare a DNA fragment. Then, a DNA fragment containing DmpR and Pₒ (see SEQ ID NO: 45) was prepared by performing amplification using a pSEVA131-phGESS4-sf plasmid (see SEQ ID NO: 44) as a template and a primer pair, pAWPS-PO(SpeI)-F and pAWPS-PO(XbaI)-R. These two DNA fragments were combined by the Gibson assembly method to construct a pAWP89S-Pₒ-dTomato plasmid (see SEQ ID NO: 46).

### 1-7. Construction of pAWP89S-Pₒ-dTomato-DmpR (Δ2-204) plasmid

In order to replace the wild-type DmpR of the pAWP89S-Pₒ-dTomato plasmid with DmpR(Δ2-204) in which the 2nd to 204th amino acids of DmpR were deleted, the pAWP89S-Ptet-dTomato plasmid was digested with SpeI/XbaI to prepare a DNA fragment. Then, a DNA fragment containing DmpR (Δ2-204) was prepared by performing amplification using the pAWP89S-Po-dTomato plasmid as a template and a primer pair, pAWPS-PO(SpeI)-F and DA-R, and a DNA fragment was also prepared by performing amplification using the pAWP89S-Po-dTomato as a template, and a primer pair, DA-F and pAWPS-PO(XbaI)-R. These three DNA fragments were combined by the Gibson assembly method to construct a pAWP89S-Po-dTomato-DmpR (Δ2-204) plasmid (see SEQ ID NO: 48).

### 1-8. Construction of pAWP89S-Pₒ-dTomato-RBS(MDH), pAWP89S-Pₒ-dTomato-RBS(MmoX), pAWP89S-Pₒ-dTomato-RBS(MmoY), pAWP89S-Pₒ-dTomato-RBS(MmoZ), pAWP89S-Pₒ-dTomato-RBS(PmoA1), pAWP89S-Pₒ-dTomato-RBS(PmoB1), pAWP89S-Po-dTomato-RBS(PmoC1), pAWP89S-Pₒ-dTomato-RBS(PmoC2), pAWP89S-Pₒ-dTomato-RBS(PmoC3) plasmids

In order to replace the RBS regulating the translation of the dTomato protein of the pAWP89S-Pₒ-dTomato plasmid with RBS(MDH), the pAWP89S-Po-dTomato plasmid was digested with PstI to prepare a DNA fragment. Then, a DNA fragment was prepared by performing amplification using the pAWP89S-Po-dTomato plasmid as a template and a primer fair, DmpR(PstI)-F and RBS-R, and a DNA fragment containing RBS(MDH) was prepared by performing amplification using the dTomato plasmid as a template and a primer fair, RBS(MDH)-F 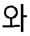 dTomato(PstI)-R. These three DNA fragments were combined by the Gibson assembly method to construct a pAWP89S-Pₒ-dTomato-RBS (MDH) plasmid (see SEQ ID NO: 49).

The pAWP89S-Pₒ-dTomato-RBS(MmoX) plasmid (see SEQ ID NO: 50) was constructed by using a DNA fragment that was amplified from a DNA sequence containing RBS(MmoX) using the pAWP89S-Pₒ-dTomato plasmid as a template and a primer fair, RBS(MmoX)-F and dTomato(PstI)-R, in the same way as above.

The pAWP89S-Pₒ-dTomato-RBS(MmoY) plasmid (see SEQ ID NO: 51) was constructed using a DNA fragment that was amplified from a DNA sequence containing RBS(MmoY) using the pAWP89S-Pₒ-dTomato plasmid as a template and a primer pair, RBS(MmoY)-F and dTomato(PstI)-R, in the same way as above.

The pAWP89S-Pₒ-dTomato-RBS(MmoZ) plasmid (see SEQ ID NO: 52) was constructed using a DNA fragment that was amplified from a DNA sequence containing RBS(MmoZ) using the pAWP89S-Pₒ-dTomato plasmid as a template and a primer pair, RBS(MmoZ)-F and dTomato(PstI)-R, in the same way as above.

The pAWP89S-Pₒ-dTomato-RBS(PmoA1) plasmid (see SEQ ID NO: 53) was constructed using a DNA fragment that was amplified from a DNA sequence containing RBS(PmoA1) using the pAWP89S-Pₒ-dTomato plasmid as a template and a primer pair, RBS(PmoA1)-F and dTomato(PstI)-R, in the same way as above.

The pAWP89S-Pₒ-dTomato-RBS(PmoB1) plasmid (see SEQ ID NO: 54) was constructed using a DNA fragment that was amplified from a DNA sequence containing RBS(PmoB1) using the pAWP89S-Pₒ-dTomato plasmid as a template and a primer pair, RBS(PmoB1)-F and dTomato(PstI)-R, in the same way as above.

The pAWP89S-Po-dTomato-RBS(PmoC1) plasmid (see SEQ ID NO: 55) was constructed using a DNA fragment that was amplified from a DNA sequence containing RBS(PmoC1) using the pAWP89S-Pₒ-dTomato plasmid as a template and a primer pair, RBS(PmoC1)-F and dTomato(PstI)-R, in the same way as above.

The pAWP89S-Pₒ-dTomato-RBS(PmoC2) plasmid (see SEQ ID NO: 56) was constructed using a DNA fragment that was amplified from a DNA sequence containing RBS(PmoC2) using the pAWP89S-Pₒ-dTomato plasmid as a template and a primer pair, RBS(PmoC2)-F and dTomato(PstI)-R, in the same way as above.

The pAWP89S-Pₒ-dTomato-RBS(PmoC3) plasmid (see SEQ ID NO: 57) was constructed using a DNA fragment that was amplified from a DNA sequence containing RBS(PmoC3) using the pAWP89S-Pₒ-dTomato plasmid as a template and a primer pair, RBS(PmoC3)-F and dTomato(PstI)-R, in the same way as above.

The primer sequences used in Experimental Example 1 are disclosed in Table 1 below.

**[Table 1]**

| Seq ID | name | sequence |
|---|---|---|
| 23 | pAW89(NotI)-F1 | agccgtgtgc gagacaccgc |
| 24 | pAW89(NotI)-R1 | cccgacaatc tagatagctg tttcctgtgt gaa |
| 25 | pAW89(HindIII)-F2 | cagctatcta gattgtcggg aagatgcgtg at |
| 26 | pAW89(HindIII)-R2 | agtctggaaa gaaatgcata |
| 27 | pAWP S (NotI)-R1 | ttcagagact agtttcgcaa cctagtgaat gag |
| 28 | pAWPS(XbaI)-F2 | ttgcgaaact agtctctgaa atgagctgtt gac |
| 29 | pAWP S (Xb aI)-R2 | tcacgcatct tcccgacaat |
| 30 | pTet-dTomato(SpeI)-F1 | tcattcacta ggttgcgaaa tcagagattt tgagacacaa |
| 31 | pTet-dTomato(SpeI)-R1 | tgctcaccat atgtatatct ccttcttaaa |
| 32 | pTet-dTomato(XbaI)-F2 | agatatacat atggtgagca agggcgagga |
| 33 | pTet-dTomato(XbaI)-R2 | tcacgcatct tcccgacaat ctacttgtac agctcgtcca |
| 34 | pAWPS-Ptrc(SpeI)-F | ctatcaacag gagtccaaga ctagtttgac accatcgaat ggtgc |
| 35 | pAWPS-Ptrc(XbaI)-R | aaagttaaac aaaattattt ctagagtgtg aaattgttat ccgct |
| 36 | pAWPS-Pm(SpeI)-F | ctatcaacag gagtccaaga ctagtacgtt cgtaatcaag ccact |
| 37 | pAWPS-Pm(XbaI)-R | aaagttaaac aaaattattt ctagacatgg tcatgactcc attat |
| 38 | pAWPS-PO(SpeI)-F | ctatcaacag gagtccaaga ctagtagcgg ataacaattt cacac |
| 39 | pAWPS-PO(XbaI)-R | aaagttaaac aaaattattt ctagatctcc aggttggcgg attgc |
| 60 | DA-R | cgcttacatg aacgacccca tcatcgagga |
| 61 | DA-F | tggggtcgtt catgtaagcg aggcccctat |
| 62 | DmpR(P stI)-F | ggccattgct gaaacctgca |
| 63 | RBS-R | tctagatctc caggttggcg |
| 64 | dTomato(PstI)-R | gatcagcgtg ccgtcctgca |
| 65 | RBS(MDH)-F | cgccaacctg gagatctaga tgcagggtcg gcatcaatca ttcttggagg agacacatgg tgagcaaggg cgagga |
| 66 | RBS(MmoX)-F | cgccaacctg gagatctaga catcattcat agaatgtgtt acggaggaaa caagtaatgg tgagcaaggg cgagga |
| 67 | RBS(MmoY)-F | cgccaacctg gagatctaga cgttcgatca acctcaaacc aaaaaggaac atcgatatgg tgagcaaggg cgagga |
| 68 | RBS(MmoZ)-F | cgccaacctg gagatctaga tggacacttt ttcaacggcc tgttaaggag aatgacatgg tgagcaaggg cgagga |
| 69 | RBS(PmoA1)-F | cgccaacctg gagatctaga gaaaataaga aaacgacaaa tttggaggta actttaatgg tgagcaaggg cgagga |
| 07 | RB S(PmoB 1)-F | cgccaacctg gagatctaga gtgacccaac agcaagaact cgaaagagga gagatcatgg tgagcaaggg cgagga |
| 17 | RB S(PmoC 1)-F | cgccaacctg gagatctaga ggcccctgtc aaccatcact ttaggaggaa caaacaatgg tgagcaaggg cgagga |
| 27 | RBS(PmoC2)-F | cgccaacctg gagatctaga ccggtgttca catcgatata acagttggag gtaaaaatgg tgagcaaggg cgagga |
| 37 | RBS(PmoC3)-F | cgccaacctg gagatctaga gtggcatcaa tccaacacca actcaggaga aatcagatgg tgagcaaggg cgagga |

### Experimental Example 2: Conjugation by triparental mating

### 2-1. Cultivation of methanotrophs (3 days before mating)

*M. capsulatus* Bath was inoculated into cell stock in 3 ml of NMS media, covered with a rubber stopper, and sealed with an aluminum seal. 50 % methane gas was supplied based on the headspace of the medium, and seed culture was performed at 37 °C, 200 rpm, and 24 hours. *M. trichosporium* OB3b strain was seed cultured in the same manner as in the *M. capsulatus* Bath, except that the culture condition was 30 °C.

### 2-2. Cultivation of methanotrophs (2 days before mating)

The *M. capsulatus* Bath was inoculated with 5 % seed in NMS media containing 10 µM CuCl₂, covered with a rubber stopper, and sealed with an aluminum seal. 50 % methane gas was supplied based on the headspace and incubated at 37 °C, 200 rpm, and 19 hours. M. *trichosporium* OB3b was subjected to main culture under the same conditions except that the culture condition was 30 °C for 30 hours.

### 2-3. Preparation of donor strain and helper strain (1 day before mating)

*E. coli* CC118 containing the pAWP89S-Pₒ-dTomato, pAWP89S-Pₘ-dTomato, or pAWP89S-P_{trc}-dTomato plasmid was inoculated in 10 ml of LB media containing Kan 5 µg/ml, respectively, and cultured at 37 °C, 200 rpm, and 19.5 hours. *E. coli* HB101, a helper strain for transformation, was cultured in 10 ml of LB media at 37 °C, 200 rpm, and 19.5 hours.

### 2-4. Mating

The OD₆₀₀ value of each cell was measured and mixed in a 50 ml tube so that the OD₆₀₀ value based on 1 ml was 3. The order of introduction was in the order of donor strain, helper strain, and methanotrophs (recipient).

After centrifugation of the strain mixture at the condition of 1000 g and 20 min, the supernatant was removed, and the strain was resuspended in 200 µl of NMS and spread on a NMS+0.12% nutrient agar plate.

### 2-5. Strains screening by single colony isolation

The spread strain was mated at 37 °C or 30 °C for 3 days, and the cells were harvested with 500µl of NMS, and diluted 10, 100, 1,000 and 10,000 times, and then, 100 µl of the cells were spread on a NMS-Kan (25 µg/ml) plate, and the transformed methanotrophs were selected. In the case of the *M. capsulatus* Bath, a single colony was streaked on an NMS-Kan (25µg/ml) plate and cultured at 37 °C for 6 days, and in the case of *M. trichosporium* OB3b, a selection process was performed by culturing the strain at 30 °C for 10 to 11 days. In the case of *M. capsulatus* Bath, transformants that were not contaminated with the donor or the helper were obtained through this selection process, and in the case of *M. trichosporium* OB3b, the selection process was repeated two more times to secure transformants through a total of three selection processes.

### Experimental Example 3: Cultivation, fluorescence analysis, and SDS-PAGE analysis of transformed methanotrophs

### 3-1. Cultivation of transformed methanotrophs

The transformed *M. capsulatus* Bath and *M. trichosporium* OB3b were inoculated by 10 % in NMS medium containing 25 µg/ml kanamycin and 10 µM CuCl₂. For the methanotrophs transformed with the pAWP89S-Po-dTomato, 10 µM of phenol was contained in the medium. After sealing, 50 % methane gas was injected based on the headspace. The transgenic *M. capsulatus* Bath was cultured at 37 °C for 24 hours or 44 to 48 hours, and the transgenic *M. trichosporium* OB3b was cultured at 30 °C.

### 3-2. Fluorescence analysis

For analysis of the expression level by fluorescence, 200 µl was sampled in a 96-well plate and a Tecan microplate reader (excitation: 535 nm, emission: 590 nm) was used. FACS analysis was performed using BD Biosciences FACSCalibur equipment (excitation: 488 nm, emission: 585 nm).

### 3-3. SDS-PAGE analysis

The cultured cells were lysed by ultrasonic waves and samples were prepared for SDS-PAGE. The protein concentration of the sample was 0.36 mg/ml in a control group not treated with phenol, and 0.30 mg/ml in the experimental group treated with 10 µM phenol.

5 % stacking gel and 15 % running gel were prepared and mounted on an electrophoresis device. In the *M. capsulatus* Bath, a sample of 5 µg/µl was loaded and electrophoresis was performed at 100V for 90 minutes. In *M. trichosporium* OB3b, a sample of 10 µg/µl was loaded and electrophoresis was performed at 100V for 110 minutes.

### Example 1: Confirmation of dTomato expression in M. trichosporium OB3b transformed with a pAWP89S-Ptet-dTomato plasmid

*M. trichosporium* OB3b transformed with the pAWP89S-Ptet-dTomato plasmid was treated with anhydrotetracycline(aTc) at 0 µM, 0.5 µM, 1 µM, or 2 µM, respectively, to induce expression, and the expression of the fluorescent protein dTomato was examined for 8 days. The experiment was performed by the method of Experimental Example 3 above.

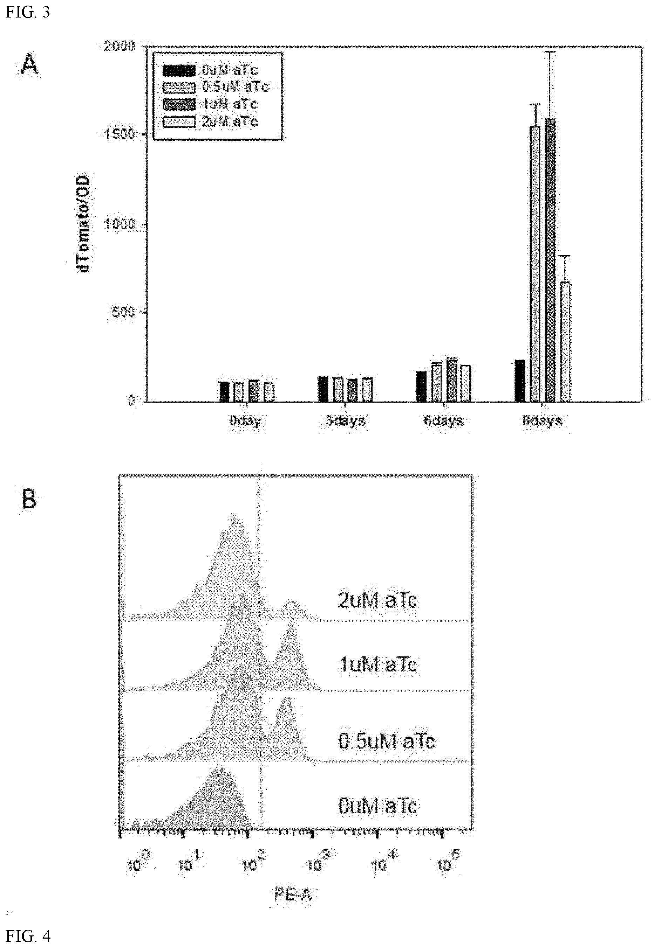

According to (A) in FIG. 3, fluorescence intensity was the highest in the groups treated with 0.5 µM and 1 µM of aTc, and rather decreased in the group treated with 2 µM. Therefore, it was confirmed that the optimal treatment concentration of aTc was 0.5 to 1 µM. In addition, the time required for expression to be most active was about 8 days.

According to (B) in FIG. 3 showing FACS analysis results, two peaks were formed based on an one-dotted chain line, which means that there are a significant number of strains expressing dTomato as well as strains that do not express dTomato. Although not clearly identified, it is considered that this is because aTc is difficult to reach the vector inside M. *trichosporium* OB3b and thus cannot uniformly induce expression of the Pₜₑₜ promoter.

### Example 2: Confirmation of dTomato expression in M. capsulatus Bath transformed with a pAWP89S-Ptrc-dTomato, pAWP89S-Po-dTomato, or pAWP89S-Pm-dTomato plasmid

### 2-1. Confirmation of expression by fluorescence measurement

The fluorescence expression of dTomato was measured in the same manner as in Example 1 above.

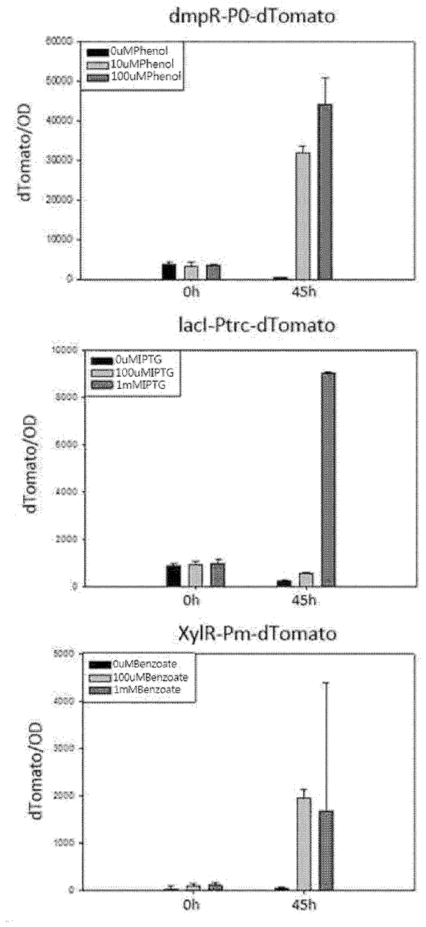

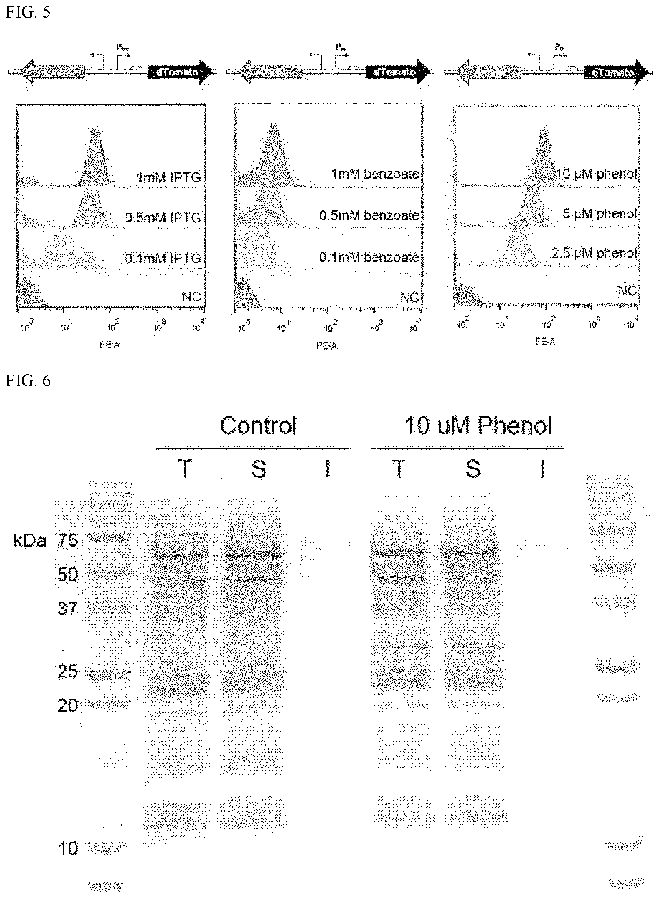

According to FIGS. 4 and 5, the *M. capsulatus* Bath into which the pAWP89S-Pₒ-dTomato plasmid was introduced showed the highest fluorescence expression value (dTomato/OD₆₀₀ₙₘ) after 45 hours compared to a group in which the pAWP89S-P_{trc}-dTomato plasmid was introduced and a group in which the pAWP89S-Pm-dTomato plasmid was introduced.

Specifically, although the strain in which the pAWP89S-Ptrc-dTomato plasmid was introduced and the strain in which the pAWP89S-Pm-dTomato plasmid was introduced were treated with a higher amount of inducer (0.1 to 1 mM IPTG or 0.1 to 1 mM benzoate) than the strain in which the pAWP89S-Pₒ-dTomato plasmid was introduced, the strains showed lower fluorescence values.

However, in the strain in which the pAWP89S-Pₒ-dTomato plasmid was introduced, it was observed that the strain growth in the group to which 100 µM phenol was treated was inhibited more than that in the strain more than the group to which 10 µM phenol was treated.

### 2-2. Confirmation of dTomato expression by SDS-PAGE

The expression level of dTomato was measured by the SDS-PAGE method of Example 3.

According to FIG. 6, a band of 26.97 kDa corresponding to dTomato, which was not seen in the control group, was clearly shown in in the experimental group treated with 10 µM of phenol. As a result of the experiment, it was confirmed that the vector containing the DmpR and Pₒ promoters induced expression of the target protein in the *M. capsulatus* Bath, which was observed by bands on the SDS-PAGE.

### 2-3. Confirmation of inhibition of leaky expression by tight regulation of M. capsulatus Bath in which a pAWP89S-Pₒ-dTomato plasmid was introduced

The *M. capsulatus* Bath in which the pAWP89S-Po-dTomato plasmid were introduced were cultured in a medium without phenol and a medium containing 10 µM phenol for 45 hours, respectively, and their dTomato fluorescence expression were compared. According to FIG. 7, assuming that the dTomato expression level in the presence of 10 µM phenol was 100 %, the rate of dTomato expression level in the absence of phenol was only less than 2 %, so it was confirmed that the inhibition of leakage expression was excellent.

### 2-4. Expression time under conditions induced by 10 µM phenol

According to FIG. 8, in the *M. capsulatus* Bath into which the pAWP89S-Pₒ-dTomato plasmid was introduced, fluorescence due to dTomato expression was observed 5 hours after phenol was supplied under the condition of supplying 10 µM phenol.

### Example 3: Confirmation of dTomato expression in M. trichosporium OB3b transformed with pAWP89S-P_{trc}-dTomato, pAWP89S-Pₒ-dTomato, or pAWP89S-Pₘ-dTomato plasmids

### 3-1. Expression confirmation by fluorescence measurement

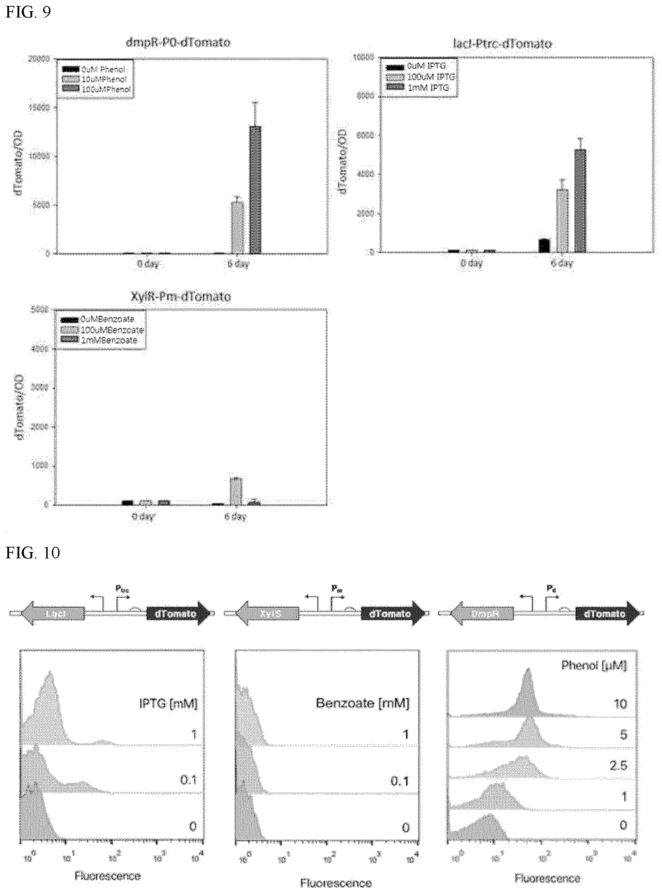

According to FIGS. 9 and 10, *M. trichosporium* OB3b introduced with the pAWP89S-Po-dTomato plasmid showed the highest fluorescence value (dTomato/OD₆₀₀ₙₘ) after 6 days than the group introduced with the pAWP89S-P_{trc}-dTomato plasmid and the group introduced with the pAWP89S-Pₘ-dTomato plasmid.

Specifically, the strain in which the pAWP89S-Ptrc-dTomato plasmid was introduced and the strain in which the pAWP89S-Pₘ-dTomato plasmid was introduced showed the lower fluorescence value (dTomato/OD₆₀₀ₙₘ) than that of the strain in which the pAWP89S-Pₒ-dTomato plasmid was introduced, even though the strains were treated with a larger amount of inducer (0.1 to 1 mM IPTG or 0.1 to 1 mM benzoate).

### 3-2. Confirmation of dTomato expression by SDS-PAGE

The *M. trichosporium* OB3b in which the pAWP89S-Pₒ-dTomato vector was introduced was subjected to the SDS-PAGE analysis under the same conditions as in 3-2 above.

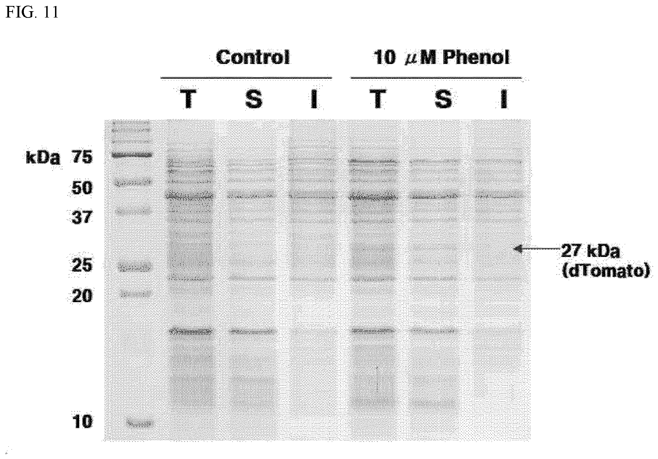

According to FIG. 11, the experimental group treated with 10 µM of phenol showed an additional band of 27 kDa corresponding to dTomato than the control group. As a result of the experiment, it was confirmed that the vector containing the DmpR and Pₒ promoters induced expression of the target protein in methanotrophs, which was observed as bands on the SDS-PAGE.

### Example 4: Confirmation of operation of a DmpR promoter by benzene

It was confirmed whether dTomato could be induced with benzene under sMMO expression conditions (copper-deficient medium).

The *M. capsulatus* Bath transformed with the pAWP89S-Pₒ-dTomato plasmid were cultured under sMMO expression condition (copper-deficient medium) and pMMO expression condition (copper-containing medium) while supplying 0.5 mM benzene for 24 hours. A strain supplied with 10 µM of phenol was used as a control group.

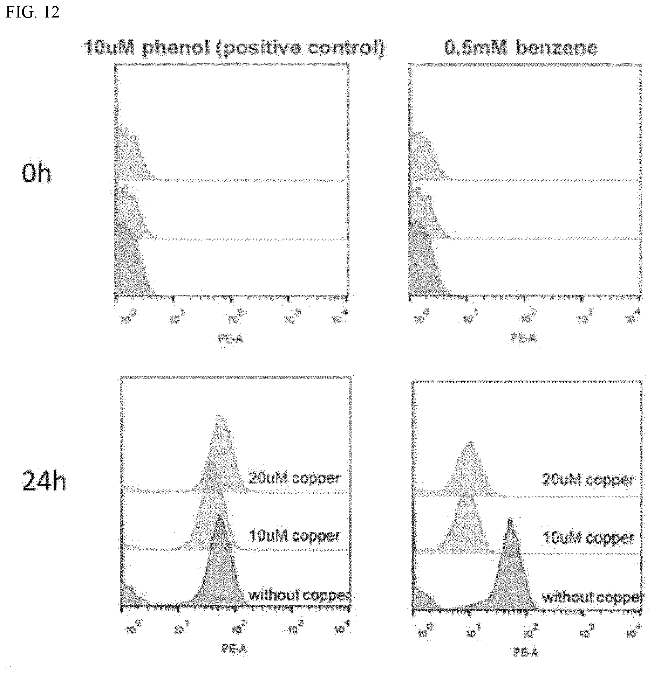

According to FIG. 12, the transformed strain cultured under the sMMO expression condition converted benzene to phenol by the sMMO enzyme activity, and dTomoto expression was highly induced as in the control group to which phenol was directly added. In the pMMO expression condition with copper, a relatively small fluorescence value was confirmed. This is an example showing that the developed expression system can be used as a sensor system that can identify the difference in sMMO and pMMO activities.

### Example 5: Regulation of protein expression by using DmpR (Δ2-204) and various RBS

To improve the expression system for methanotrophs of the disclosure, a constitutive expression system was prepared by removing A domain of DmpR. In addition, in order to regulate the expression level of the protein in various ways, various ribosomal binding sites derived from methanogens were combined with the Pₒ promoter to identify the expression level of the dTomato protein.

Specifically, a Pₒ promoter expression plasmid in which DmpR was replaced with DmpR (Δ2-204) in which the 2nd to 204th amino acids corresponding to the domain of DmpR were deleted and an expression plasmid in which an existing T7 gene 10 ribosomal binding sites were replaced with the ribosomal binding sites derived from *M. capsulatus* Bath genes, *MDH, MmoX, MmoY, MmoZ, PmoA1, PmoB1, PmoC1, PmoC2* or *PmoC3* were constructed. Thereafter, the *M. capsulatus* Bath transformed with each plasmid was cultured for 45 hours under the condition of supplying 0 µM or 10 µM phenol, and then the fluorescence of dTomato proteins was compared.

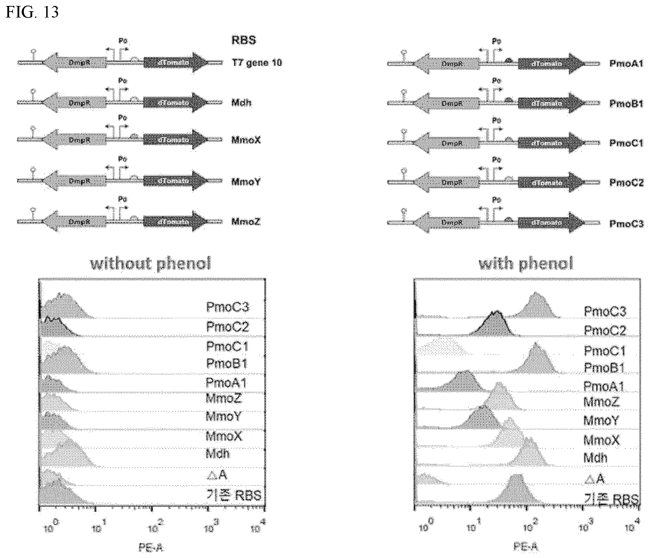

According to FIG. 13, unexpectedly, when the A domain of DmpR was removed, dTomato protein was not expressed at all in methanotrophs even when phenol was added.

In the case of using various ribosomal binding sites derived from methanotrophs, the expression level of dTomato in using the ribosomal binding sites derived from MDH, PmoB1, and PmoC3 was higher than that in using the existing T7 gene 10, and in particular, the fluorescence value in using PmoB1 was 2.3 times higher than that in using the existing T7 gene 10. In addition, the dTomato fluorescence value in using MmoX, MmoY, MmoZ, MmoC1, and MmoC2 ribosomal binding sites was lower than that in using an existing ribosomal binding site, but it was confirmed that the expression intensity was diverse. In addition, it was confirmed that the fluorescence values at the single cell level were uniformly distributed in all the results.

### Example 6: Confirmation of industrial usefulness (FmDTA expression in M. capsulatus Bath)

A constitutive pTac promoter-based (FmDTA-pAWP) or Pₒ promoter-based (Po-FmDTA-pAWP) expression system was constructed to introduce high-purity pharmaceutical raw material, D-threonine production enzyme (threonine aldolase derived from *Filomicrobium marinum* (FmDTA)) into methanotrophs.

As a result of culturing methanotrophs containing each plasmid and analyzing the expression level by SDS-PAGE, it can be confirmed that the expression level is remarkably high when the Po system is used. As a reference, the band at the same position cannot be observed as expected in a negative control, methanotrophs containing a pAWP89 empty vector. This is an example showing that the newly constructed Po system is useful for high expression of an enzyme of interest in methanotrophs, and is expected to be introduced and utilized in various enzymes.

### Example 7: Use of CRISPR/Cas9-based cytosine base editor in methanotrophs

To verify the activity of a cytosine base editor using the Pₒ promoter in methanotrophs, a nCas9-BE(APOBEC-nCas9-UGI) plasmid and a pAWPSG-Pₒ-nCas9-BE-RiboJ-sgRNA(McglgA4) regulating sgRNA expression with the Pₒ promoter plasmid (see SEQ ID NO: 58) were constructed. In addition, to enhance sgRNA expression, a pAWPSG-Pₒ-nCas9-BE-pTac-RiboJ-sgRNA(McglgA4) plasmid (see SEQ ID NO: 59) was constructed by inserting a Ptac promoter in front of the sgRNA. 5'-GTTTCCAGCCGAGGCCGAGC-3' was selected as the sgRNA sequence, and this sgRNA sequence binds to the glycogen synthase gene *glgAl* to induce mutation of the TGG(Trp) codon to a stop codon. Thereafter, *M. capsulatus* Bath transformed with the constructed cytosine base editor plasmid was cultured in the presence of phenol, and the target sequence of *glgAl* was analyzed by Sanger sequencing. In addition, in order to accumulate the mutation of the target sequence, subculture of the strain was performed a total of three times.

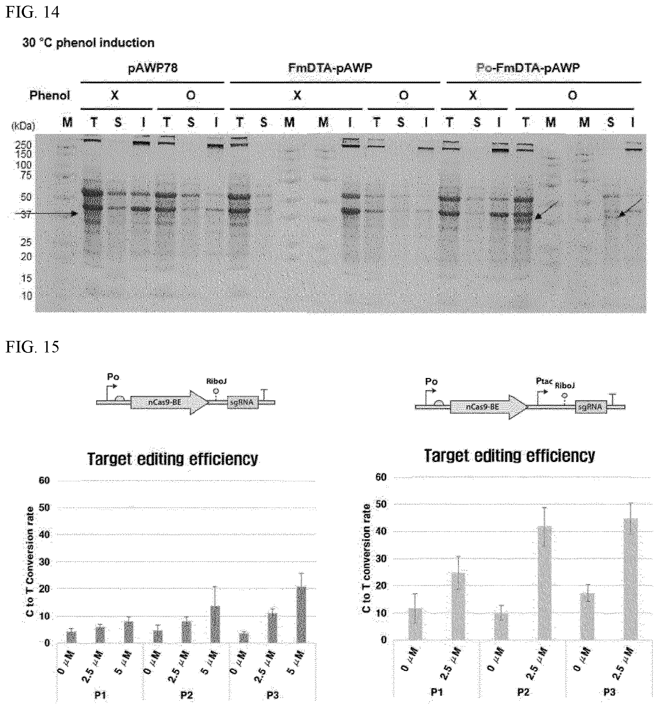

As a result, as shown in FIG. 15, it was confirmed that the target sequence was edited with thymine in more than 40 % of the total cells when using the cytosine base editor system. In addition, it was found that the editing was performed more efficiently when the tac promoter was placed in front of the sgRNA, and it was confirmed that the point mutation was accumulated as subculture was performed. Through these results, it was confirmed that the cytosine base editor system using the Pₒ promoter operated well in methanotrophs, and the productivity of target substances could be expected to increase through the inhibition of glycogen biosynthesis.

The preferred embodiments of the disclosure have been explained so far. A person skilled in the art will understand that the disclosure may be implemented in modifications without departing from the basic characteristics of the disclosure. Accordingly, the described embodiments should be considered in descriptive sense only and not for purposes of limitation. The scope of the disclosure is defined not by the detailed description herein but by the appended claims, and all differences within the scope will be construed as being included in the present inventive concept.

## Claims

1. A vector for transfer and utilization in a methanotroph, comprising:
a first expression cassette comprising DmpR as a transcriptional regulator and a promoter operably linked thereto; and
a second expression cassette comprising a sequence encoding a target polypeptide and a Pₒ promoter operably linked thereto.

2. The vector of claim 1, wherein the target polypeptide is a marker, a reporter, a polypeptide capable of improving production of a desired metabolite, or a polypeptide capable of promoting growth of the methanotroph.

3. The vector of claim 1, wherein the expression of the target polypeptide is induced by a phenolic compound.

4. The vector of claim 1, wherein the first expression cassette and the second expression cassette are arranged in the same or opposite transcriptional directions.

5. The vector of claim 1, wherein the vector is a plasmid.

6. The vector of claim 1, wherein the methanotroph is selected from the group consisting of *Methylomonas sp., Methylobacter sp., Methylococcus sp., Methylosphaera sp., Methylocaldum sp., Methyloglobus sp., Methylosarcina sp., Methyloprofundus sp., Methylothermus sp., Methylohalobius sp., Methylogaea sp., Methylomarinum sp., Methylovulum sp., Methylomarinovum sp., Methylorubrum sp., Methyloparacoccus sp., Methylosinus sp., Methylocystis sp., Methylocella sp., Methylocapsa sp., Methylofurula sp., Methylacidiphilum sp., Methylacidimicrobium sp.,* and *Methylomicrobium sp.*

7. The vector of claim 1, wherein the methanotroph is *Methylococcus capsulatus* Bath *or Methylosinus trichosporium* OB3b.

8. A methanotroph in which the vector of claim 1 has been introduced.
